# EUROPEAN PATENT SPECIFICATION

(11) **EP 2 035 018 B1**
(45) Date of publication and mention of the grant of the patent: **22.10.2014**
(21) Application number: 07719893.5
(22) Date of filing: 04.06.2007
(51) Int. Cl.: A61K 33/04, A61K 47/30, A61K 9/14, A61P 31/00

(54) **Use of micron-sized sulphur for the prevention and treatment of pathogenic disorders in humans and animals**
Verwendung von Schwefel in Mikrogrösse zur Verbeugung und Behandlung von Krankheiten in Menschen und Tieren
Soufre microdimensioné pour la prevention et le traitement de maladies chez les humains et les animaux

(30) Priority: 02.06.2006 US 803731 P; 23.06.2006 US 805619 P
(43) Date of publication of application: 18.03.2009
(73) Proprietor: Natural MA Inc., Calgary, Alberta T2R 1J8 (CA)
(72) Inventor: WALKER, Ralph, Byemoor, AB T0J0L0 (CA)
(74) Representative: Vossius & Partner
(86) International application number: PCT/CA2007/000977
(87) International publication number: WO 2007/140588

(56) References cited:
- WO-A-00/43021
- WO-A-97/48403
- CA-A1- 2 265 926
- DE-A1- 19 934 169
- FR-A- 2 530 925
- US-A- 5 997 892
- SWEETMAN S.: '"Sulfur", Martindale: The Complete drug reference', vol. 33RD ED., PHARMACEUTICAL PRESS, LONDON pages 1123 - 1124, XP008097337
- BOERICKE W. ET AL.: 'Pocket Manual of Homeopathic Materia Medica and Repertory', 1994, B. JAIN PUBLISHERS, NEW DELHI pages 620 - 623, XP008097150
- LIN ET AL.: 'Sulfur revisted' J. AM. ACAD. DERMATOL. vol. 18, no. 3, March 1988, pages 553 - 558, XP002966196
- STARKS ET AL.: 'Response of lambs fed varied levels of elemental sulfur, sulfate sulfur and methionine' J. ANIM. SCI. vol. 13, 1954, pages 249 - 257, XP008091434
- SHAVER ET AL.: 'Feeding Dairy Cows for Efficient Reproductive Performance', [Online] 1997, XP008095415 Retrieved from the Internet: <URL:http://www.oznet.ksu.edu/library/lvstk 2/samplers/ncr366.asp>
- DIALOGUE/NEWSLETTERS: 'Approved food contact uses of lignosulfonates', [Online] vol. 6, no. 1, September 1997, pages 1 - 3, XP008092415 Retrieved from the Internet: <URL:http://www.lignin.org/dialogue.html>

## Description

### BACKGROUND OF THE INVENTION

Sulphur is a non-metallic element that occurs either free or combined with other elements, especially in sulphides and sulphates. Sulphur is an absolute dietary requirement as both amino acids, methionine and cysteine contain sulphur. Typically, most dietary sulphur is in the form of protein.

While elemental sulphur is non-toxic, many simple sulphur derivatives are, such as sulphur dioxide and hydrogen sulphide. Contact can cause eye, nose, throat, skin and lung irritation, the severity of which is directly dependent on the duration and concentration of exposure. Common oxidization states of sulphur include -2, +2, +4 and +6. The redox and reactive character of the sulphur combined with that of the transition metal, leads to versatile chemistry that has potentially important biological implications for many diseases. Sulphur has been recognized since ancient times to have a myriad of medicinal properties, however, in large parts, these properties remain to be substantiated through modem scientific methods. Sulphur in it's native solid state ordinarily exists as cyclical crown-shape S8 molecules. However, many other rings have been prepaired, including S7, S12, and S18. The S8 rings are stable atomic molecules with the sulphur atoms strongly bonded to each other. This gives elemental sulphur a relatively water-inert existence.

Application FR-A-2 530 925 describes the use of a composition comprising sulphur and lignin sulfonate with a particle size of less than 10 microns as a fungicide.

Application DE 199 34 169 describes a pharmaceutical composition comprising particles of sulphur of a diameter of less than 1.5 microns as an antibacterial compound.

WO 97/48403 describes a pharmaceutical composition comprising finely divided sulphur for use in the treatment of diseases such as Lupus erythematosus. WO 00/43021 describes a pharmaceutical composition comprising an ultrafine powder of sulphur for use in the treatment of, amongst others, rheumatoid arthritis. US-A-5 997 892 describes the use of sublimed/powdered sulphur in medicine. A combination with sodium lignin sulfonate is not described.

Sodium lignin sulfonate is a break down product of lignin, one of the essential constituents, along with cellulose, providing the structure of wood. Lignin is an amorphous polymer made of the building units trans-coniferyl alcohol, trans-sinapyl alcohol and trans-p-coumaryl alcohol. Lignin sulfonates are short chain breakdown products resulting from the sulfite pulping process used to delignify wood. They are heterogeneous in nature, with the proportion of each of the propanoid phenolic alcohols of lignin depending on the species of wood used in the pulping process. Sodium lignin sulfonate as a product contains many compounds besides the sulfonated hydrolysis products, including sugars and saccharides, hydrolysis products of fatty acids and other organic compounds, and some minerals. Most of the sulfonic acid groups introduced into the lignin replace hydroxylated substituents at the alpha carbon of the propane side chain. Free phenolic structures are rapidly sulfonated (Gargulak, J.D. and Lebo, S.E. (2000), Commercial Use of Lignin-Based Materials, in "Lingin: Historical, Biological and Materials Perspectives, Glasser, W.G., Northey, R.A. and Schulz, T.P., eds., ACS Symposium Series 742, Ch.15).

The medicinal properties of sodium lignin sulfonates have not been extensively studied. However, they are purported to have value as antiviral agents and also as antithrombotics. Linin sulfonates are accepted as being non-toxic at typical use levels, and this is supported by a government of Denmark report (Toblassen, L.S., Nielsen, E., Norhede, P. and Ladefoged, O. (2003) Appendices 1-18 to : Report on the Health Effects of Selected Pesticides Coformulants, Working Report No. 51, 2003, Danish Environmental Protection Agency). The US FDA has a list of products that are generally recognized as safe. Sodium lignin sulfonate is on the list and has GRAS status for indirect exposure to humans. It is also approved by the USDAfor inclusion in food animal feeds.

### SUMMARY OF THE INVENTION

According to a first aspect of the invention, there is provided a pharmaceutical composition comprising as an active ingredient sulphur particles less than 300 microns and sodium lignin sulfonate, as characterized in the appended claims.

According to a second aspect of the invention, there is provided a method of preparing a pharmaceutical composition comprising admixing an effective amount of sulphur particles less than 300 microns with sodium lignin sulfonate, as characterized in the appended claims.

The inventor of said equilibiotic compound believes that a common link in many disorders of animal and mankind alike has been discovered. This common link of pathogenic disorders can be effectively treated or prevented or ameliorated with the proper administration of said equilibiotic compound. While not wishing to be bound to a particular hypothesis, the inventor believes that the SLS catalyst systemically frees up the sulphur atoms to be oxidized, resulting in a systemic anti-oxidant. This anti-oxidant property weakens or attenuates the replication cycle of the particular pathogen. In turn, a corresponding reduction reaction frees up the hydrogen proton. In essence, the equilibiotic compound achieves systemic anti-oxidant and systemic hydrogen proton therapy without deleterious side effects. Once again the body can establish equilibrium or homeostasis from a pathological imbalance.

### DESCRIPTION OF THE PREFERRED EMBODIMENTS

Unless defined otherwise, all technical and scientific terms used herein have the same meaning as commonly understood by one of ordinary skill in the art to which the invention belongs. Although any methods and material similar or equivalent to those described herein can be used in the practice or testing of the present invention, the preferred methods and materials are now described.

As used herein, 'animal' refers to a vertebrate and/or an animal having a circulatory system. As such, as used herein, 'animal' refers to for example but by no means limited to humans, swine, bison, cattle, dogs, cats, chickens, turkeys and the like.

On a global level the human population has more than quadrupled in the last 60 years, growing from roughly 1.5 billion to 6.5 billion from 1950 to date. Obviously, modem medicine as we know it has been very effective.

However, in the last decade or more we have seen a great increase in the disorders mentioned previously in this application. If we look at our general approach to these disorders, we see a common thread of modem treatment. This thread is one based on killing the pathogens causing the disorders. For example, antibiotics to kill the bad bacteria; antivirals to kill the bad viruses; vaccines prepared by killing the viruses and bacteria; chemo and radiation therapy to kill the cancer cells. Furthermore, today's common medical approach to organ disorders has been to not regenerate functioning of said organ but rather let the organ die while we replace the organ's function. For example, with diabetes, we presently give insulin therapy as opposed to having the pancreas do it's job. Meanwhile the untreated pancreas dies.

Described herein is a method of not killing but one of restoring balance or equilibrium from a pathological imbalance or disorder for all animals including humans. From our broad based findings, it is clear we have found a common link in preventing and treating many medical disorders and infectious diseases. This common link is herein defined as pathogenic attenuation via an equilibiotic compound.

As discussed herein, an 'equilibiotic compound' refers to a compound or composition capable of re-establishing a healthy equilibrium or homeostasis from an imbalance caused by a pathogen or disorder. In a preferred embodiment, as discussed herein, the equilibiotic compound comprises micron-sized sulphur, as characterized in the appended claims. It is of note that as used herein, 'micron-sized sulphur' refers to sulphur particles of less than 300 microns for example less than 75 microns or 74 microns or less, more preferably to a preparation wherein 95-98% of the particles are 44 microns or less or less than 45 microns. As discussed below, such preparations are commercially available. As will be appreciated by one of skill in the art and as discussed below, while not wishing to be bound to a specific theory, it is believed that the particle size directly influences effectiveness and accordingly smaller particles are likely to be more effective. As will be appreciated by one of skill in the art, '300 microns' refers to the size of the sulphur particle and given that all particles may not be perfectly spherical, 'diameter' in its strictest sense is not necessarily the proper term but may be used herein for illustrative purposes.

In a preferred embodiment, the equilibiotic composition comprises a mixture of sulphur particles of 74 microns or less as defined above and sodium lignin sulfonate.

In a further embodiment of the invention, there is provided a method of preparing a pharmaceutical composition comprising mixing an effective amount of sulphur particles of 300 microns or less, for example, 74 microns or less or at least 74 microns or less than 75 microns sodium lignin sulfonate. As discussed below, the pharmaceutical composition is formulated for oral or injectable administration. As will be appreciated by one of skill in the art, 'an effective amount' will of course depend on many factors, including but by no means limited to the age, weight and general condition of the animal as well as the pathogen or disorder, as discussed below. It is of note that examples of suitable effective amounts under certain conditions are described herein.

As described herein, the administration of a pharmaceutical composition (equilibiotic composition) comprising an effective amount of micron-sized sulphur as defined herein has many purposes and many beneficial outcomes, as discussed below. In general, it may be considered as a method of improving general health of an individual comprising administering an effective amount of a pharmaceutical composition comprising micron-sized sulphur as described above. As used herein, 'pharmaceutical composition' refers to a composition administered for a therapeutic or medicinal purpose but also to a composition administered as part of a feed ration or as a vitamin or supplement as discussed below, that is, arranged for administration to an animal, either orally, intravenously, IP, IN or the like, as described below.

As discussed below, 'general health' can be measured in a number of ways, for example, by feeding efficiency, by general feeling of well-being or by treatment, amelioration or lessening of symptoms associated with a pathogen, pathogenic state, disease or disorder. Examples include but are by no means limited to the following: A) lung and airway disorders B) digestive disorders C) hormonal disorders D) cancer E) viral infections. In general, a 'pathogenic state' may be considered to be any state wherein abnormal functioning is occurring, whether the pathogen is foreign (viral or microbial) or not. As discussed below, pharmaceutical compositions as described herein may be used to treat these conditions.

In a typical livestock operation, considerable losses are incurred due to viral and/or bacterial infections. It is important to note that these losses extend beyond mortality rates, as livestock which are ill typically have reduced feeding and reproductive performance. Similarly, unicellular and multi-cellular parasites such as cryptosporidium, mange-lice, mites, worms and fungal infections such as foot rot also negatively impact general health and feeding and reproductive performance.

Described herein is a method of improving general health of animals comprising administering to an animal in need of such treatment an effective amount of equilibiotic compound as described herein. Also described is a method of increasing the rate of weight gain or feeding performance of an animal, a method of treating or preventing or ameliorating a pathogenic infection in an animal in need of such treatment, a method of increasing rate of conception, birth rates, milk production in an animal and other benefits related to the administration of an effective amount of the equilibiotic compound or composition as discussed below. As discussed below, 'feed performance', 'reproductive performance' and the like can be measured by comparing a treatment group with a non-treatment group, as shown in the examples.

As used herein, 'equilibiotic compound' refers to natural-raw elemental (earth-formed) sulphur that has been reduced in particles size to particles that are less than 300 microns. In addition, what is termed as a natural occurring catalyst compound (from the bark of trees) known as sodium lignin sulfonate (abbreviated SLS) is added. While not wishing to be bound to a particular theory or hypothesis, it is hypothesised that SLS acts as a catalyst whereby sulphur atoms are taken off of their atomic ring structures and function as a major systemic antioxidant. In turn, these sulphates (eg. calcium sulphate, sodium sulphate) from the oxidization of sulphur are expelled with normal urination and defecation. With this systemic oxidization reaction a corresponding systemic reduction reaction takes place. In turn, this systemic reduction reaction produces hydrogen protons systemically. The goal of the equilibiotic treatment is to re-establish normal balances or equilibrium states of both the fundamental elements of oxygen and hydrogen proton systemically. In this way, the infections, diseases and disorders previously mentioned are effectively prevented and treated. While not wishing to be bound to a particular theory, the equilibiotic treatment is in essence depriving the pathogens of oxygen and increasing the hydrogen proton concentration creates an inhospitable environment for pathogenic replication whereby self immunization, self containment and self rejection of pathogens are completed.

Therefore, as used herein, an equilibiotic is a naturally occurring element and/or compound, that when properly administered, is capable of re-establishing a healthy equilibrium or homeostasis from a pathological imbalance or disorder. The particular equilibiotic compound described herein acoomplishes it's task by depriving the pathogen of oxygen with available sulphur atoms that are oxidized. Meanwhile, complete attenuation of pathogenic replication cycles is accomplished with increased or normalized hydrogen protons from a corresponding reduction reaction.

Methods for manufacturing micron-sized sulphur are well-known in the art. One such method is described in US Patent 5,788,896, which discloses the preparation of various sizes of micron-sized sulphur. Specifically, this patent teaches a method of preparing micron-sized sulphur wherein sulphur granules, the vast majority of which are of a size less than 45 microns, are produced. It is of note that condition for optimizing production of smaller sized particles are also described in this patent. It is further of note that other methods may be used or may be developed for the production of micron-sized or nano-sized sulphur particles and are within the scope of this invention, as discussed herein.

As discussed above, the micron-sized sulphur may be derived from a commercial product available which produces particles as follows: 100% are 74 microns or less and 98% are 44 microns or less. This product also contains approximately 5% to 16% sodium lignin sulphonate which acts as a catalyst removing the sulphur atoms off of it's ring structure thereby making the sulphur atoms available for systemic oxidization with a corresponding reduction for the production of systemic hydrogen protons. Also described herein is that other forms of lignin sulphonate may be added, for example, the calcium salt, or other suitable agents may be added. It is of note however that the process used commercially can produce particles to 5 micron in size, the significance of which is discussed in greater detail below. It is also of note that at present, the product made by the process is in granular form and fines below the 200 mesh size are removed. However, the micron-sized sulphur does not necessarily have to be in a granular form. Alternatively, granules could be ground into a powder form. As discussed herein and as will be apparent to one skilled in the art, in some embodiments, the powdered form is a preferred method of administration. Simple grinding of the granular form can produce the powdered form.

As described herein, an effective amount of equilibiotic compound is administered to an animal in need of such treatment, either orally or injected. As will be appreciated by one of skill In the art, 'an effective amount' of equilibiotic compound is an amount that is sufficient to achieve the desired result. As discussed herein, in most embodiments, the desired result is the attenuation of the pathogen, for example, fungi, cancer cells, viruses and/or bacteria. It is of note that the 'effective amount' may vary in accordance with the type of animal, age, weight, general condition and mode of administration. Moreover, the effective amount for pathogenic attenuation may be very pathogenic specific. For example, a unicellular parasite such as cryptosporidium (which is physically larger and more complex) may require a significantly higher effective dosage level that most bacterial or viral infections.

As used herein, 'pathogenic attenuation' refers to a slowing down or decrease in the rate of replication or reproduction of the pathogen. While not wishing to be bound to a particular hypothesis, the inventor believes that administration of an effective amount of the equlibiotic compound to an animal in need of such treatment creates an inhospitable environment for the pathogen which in turn leads to attenuation of the pathogen within the host animal. As will be appreciated by one of skill in the art, attenuation of the pathogen enables the host animal's defences, for example, the host's immune system, to more quickly and effectively eliminate or control the pathogen. As will be appreciated by one of skill in the art, activation of the host's natural defences against pathogens often involves an elevation of body temperature, with an increase of inflammation which can have many deleterious effects on many other aspects, for example, but by no means limited to nutritional and digestive performance and reproduction, for example, ovulation and ejaculate volume as discussed below. Similarly, pathogenic infections can reduce many physiological performances even if no symptoms of the pathogenic infections are noticeable or detectable. As such, attenuation of the pathogens will result in less prolonged activation of the host's defences such as the immune system which will in turn improve physiological performance such as reproductive performance as discussed below and as demonstrated in the examples.

As will be appreciated by one of skill in the art, as used herein, 'treating' in all of its grammatical forms, does not necessarily require that the animal in need of such treatment have visible symptoms. For example, an animal may be infected with a microbial pathogen that causes pneumonia but because of the administration of an effective amount of equilibiotic compound the microbial pathogen may not reach the levels within the host necessary for visible symptoms to be noticed although for example core body temperature may be elevated somewhat and inflammation incurred. As discussed above, the effect of these treatments thus may be seen in increased or improved physiological performance or increased reproductive performance compared to an animal of similar age, sex and weight not administered an effective amount of the equilibiotic compound or mock administered or control-treated. As discussed herein, increased feeding performance can be measured by rate of weight gain as well as other parameters known in the art. Similarly, reproductive performance can be measured by for example birth size, ejaculate volume, frequency of conception and the like. It is of note that while 'improved general health' may be considered to be a more subjective term, this can also be measured by comparing the appearance of an animal administered an effective amount of equilibiotic compound to a control animal of similar age, sex and weight which has not been administered equilibiotic compound.

For example, in some embodiments, animals in need of treatment are administered an effective amount of equilibiotic compound as part of a nutritional and/or supplemental regime. In these embodiments, the equilibiotic compound is administered to the animal orally, as part of a nutritional ration, dissolved or suspended in drinking water, mixed in with a mineral supplement, as an orally administered powder or capsule, or injected intramuscularly or intravenously or subcutaneously. As will be appreciated by one skill in the art, methods for the manufacture of powders, injectable solutions and capsules for human consumption are well-known in the art and are within the scope of the invention.

As discussed above, in a preferred embodiment, the equilibiotic compound is a commercial product wherein 100% of the sulphur particles are 74 microns or less and 98% or 95% are 44 microns or less. Aforementioned, SLS acting as a catalyst is included at a rate of 5 to 16%.

In some embodiments, equilibiotic compound wherein at least 95% of the particles are 44 microns or less is administered to an animal in need of such treatment at 0.5-1.5% of a nutritional ration (w/w) or in drinking water (w/v). There is a pathogenic specificity whereby larger more virulent pathogens require higher dosage levels to acquire effective pathogenic attenuation to re-establish equilibrium from a pathological imbalance.

In other embodiments, an animal in need of such treatment is administered 5.0ml to 10ml per 100 pounds of body weight of a saturated solution of equilibiotic compound. The saturated solution is prepared by adding the equiliboitic compound to distilled warm water at body temperature until saturation is reached. For example, in an 8 oz solution in warm distilled water, 2 oz of the equilibiotic compound is added. As will be appreciated by one of skill in the art, in other embodiments, a larger amount of a more dilute solution may be administered. It is of note that provided the amount of the dilute solution was sufficient to accomplish at least one of the following: improve feeding performance, improve reproductive performance, and improve general health without being toxic to said animal, it is an effective amount of equilibiotic compound. While not wishing to be bound by a particular theory, the inventor believes unlike raw sulphur the micron-sized sulphur has considerable surface area which promotes systemic absorption of the sulphur atoms by the animal.

As will be appreciated by one of skill in the art, micron-sized sulphur having a smaller average size will have greater surface area and will be absorbed even more readily. As such, it is important to note that the above values are for specific micron-sized sulphur and lower amounts of smaller sulphur particles may be used. As discovered by the inventor, in fact, when comparing to larger sized sulphur particles (ie: 300 microns) the smaller sized sulphur particles (ie: 95% at 44 microns or less) was far more effective in preventing and treating of above mentioned disorders. Logically, smaller micron-sized particles of sulphur all the way down to nano-sized particles of sulphur will likely be more effective in preventing and treating the above mentioned infections and disorders. Accordingly, it is to be understood that 'an effective amount of micron-sized sulphur' as used herein refers not only to the specific values given for the equilibiotic compound or similar sized products but also to biological equivalents thereof, that is, smaller sulphur particles which may be used at lower concentrations or amounts and are within the scope of the invention.

It is of relevance to mention two other common medical observations.

Firstly, the normal openings or apertures (ie: mouth, ears, nose, eyes, anus, vagina, and penis openings) of all animals to the external environment have a higher hydrogen proton concentration (lower pH) than the internal body. (ie: blood pH) In fact, it has been shown that the lower pH attenuates the pathogen as it enters the host body. Secondly, most immunizations of man made vaccines are really just attenuated concentrations or amounts of pathogens. (eg. viruses or bacteria).

Further described herein is a method of treating or preventing or ameliorating a pathogenic infection comprising administering to an animal in need of such treatment an effective amount of equilibiotic compound. As such, administration of an effective amount of equilibiotic compound to an animal in need of such treatment will have at least one of the following effects: reduction of severity or elimination of symptoms associated with pneumonia (fever, inflammation, difficulty breathing etc), reduction of severity or elimination of symptoms associated with diarrhea, reduction of severity or elimination of symptoms associated with fungal, viral and/or bacterial infections as discussed herein and as known in the art and the like.

As will be appreciated by one of skill in the art, microbial infections include bacterial infections and viral infections. Examples of micro-organisms that cause microbial infections include but are by no means limited to E. coli, Salmonella, Campylobacter, avian flu virus, PCV2, mycoplasm, PRRS, PIRAL, IBR, Streptococci and fungal infections. As will be appreciated by one of skill in the art, this list is for illustrative purposes only and is by no means exhaustive and many other suitable microbial pathogens, will be well known to those of skill in the art.

Also described herein is a method of improving feed performance comprising administering to an animal in need of such treatment an effective amount of equilibiotic compound. As will be appreciated by one of skill in the art, administering an effective amount of equilibiotic compound will accomplish at least one of the following: improving rate of weight gain, improving feed consumption and the like.

Further described herein is a method of improving reproductive performance comprising administering to an animal in need of such treatment an effective amount of equilibiotic compound. As will be appreciated by one of skill in the art, administration of an effective amount of equilibiotic compound will accomplish at least one of the following: increase litter size, increase sperm volume and the like.

The invention will now be described by way of examples.

### Example 1 - Administration of Equilibiotic compound to Swine.

Feeders weighing approximately 65 pounds were purchased and fed to a weight of 175 pounds. Antiviral vaccines, antiparasitic treatments, and antibiotics were administered at the time of receipt and also during periods of stress and/or sickness. Traditionally, the following health problems have been incurred by newly arriving feeders: viral, parasitic and bacterial infections resulting in pneumonia-related death, bacterial infections resulting in diarrheal death (dehydration) and parasites which reduce general health and feeding performance of animals. Viral and bacterial infections usually begin after 10 days and last up to 30 days after arrival if survive.

For example, prior to administration of equilibiotic compound, 2,560 swine were purchased. Of these, 226 died of pneumonia, 256 died of diarrhea (scours) and 53 did not thrive and died from what was effectively a wasting disease (with parasitic worms and mange being visible signs thereof) prior to reaching the target weight of 175 pounds. As will be apparent to one of skill in the art, this represents approximately a 21% mortality rate. Furthermore, considerable ongoing maintenance of the herd was required to treat animals which became ill. This in turn impacted feeding performance as it took approximately 6 months for the remaining animals to reach the target weight of 175 pounds.

A herd of 2,340 swine were subsequently administered an effective amount of equilibiotic compound for example, 0.5%-1.5% of equilibiotic compound in feed and/or drinking water. As discussed below, animals which still developed pneumonia or diarrhea-associated symptoms were administered equilibiotic compound as discussed below. Of these 2,340, 10 died of pneumonia, 8 died of scours and 1 died of wasting disease which is a mortality rate of less than 1% (0.7%). Furthermore, the target weight of 175 pounds was reached in approximately 3 and a half months, clearly indicating that feeding performance and efficiency was increased as less feed and less time was required to meet the target weight.

Thus administration of an effective amount of micron-sized sulphur had a significant impact as the mortality rate was dramatically reduced (less than 1% from 21 %) and feeding performance was greatly increased (approximately 110 days to reach 175 pounds compared to approximately 180 days previously). This represents a significant improvement in the efficiency and profitability of the operation as there are fewer losses and a greatly reduced handling time.

### EXAMPLE 2 - REPRODUCTIVE PERFORMANCE IN SWINE

In a separate experiment, a farrowing operation comprising of approximately 250 Durrock guilts were administered an effective amount of equilibiotic compound as described above. The inclusion rate of the equilibiotic compound was 0.75% of complete feed. The equilibiotic compound was also included in solution in the mild replacement pipeline in the crates for the suckling pigs at the rate of 2 tablespoons per gallon.

Following commencement of the equilibiotic compound treatment regime, it was found that sperm ejaculate volume for artificial insemination increased by 50-100%. Furthermore, the success rate for artificial insemination increased from an average of 57% (8/14) to 93% (13/14) on a weekly breeding basis. Births per litter also increased from an average of 7.5 to 8 piglets per litter to approximately 10 per litter. Weaning weight also increased from an average of 8-10 pounds to 13-14 pounds.

Furthermore, prior to administration of the equilibiotic compound, 6 of 12 swine died due to pneumonia while 90 guilts died. Following administration of the equililbiotic compound as discussed above, no boars or guilts were lost to pneumonia.

As can be seen, administration of the effective amount of results in more piglets being born (13 successful inseminations X 10 piglets per litter = 130 piglets compared to 8 successful inseminations X 8 piglets per litter = 64 piglets) which have improved feeding performance as evidenced by the increased weaning weight. Thus, more piglets can be raised in the same period of time.

It is of note that prior to the commencement of the equilibiotic compound treatment, the above mentioned farrowing operation was confirmed with the diseases of micoplasma pneumonia and lepto/parvo diseases. Equally of note, previous antibiotic and viral vaccinations were non-effective.

### EXAMPLE 3 - IM INJECTION

In this experiment, a cow suffering from pneumonia was injected with 8 ml per 100 pounds body weight of a saturated solution of equilibiotic compound wherein at least 95% of the particles were 44 microns or less, and including the catalysts SLS as discussed above.

Specifically, prior to administration, the animal had an elevated body temperature (42°C), laboured breathing (gasping), dehydration, weakness and was unconscious and non-responsive. In fact, the said cow's body temperature was monitored at 42°C for twenty five minutes and one could flick a finger at said cow's eye with no reflex movement or closing of eyelid.

Following administration of an effective amount of the equilibiotic compound as described above by injection in the neck muscles, within one hour, the animal was standing and responsive, the severity of the symptoms had lessened and the body temperature had lowered to 38°C. It is of note that this experiment has been repeated 14 times (9 times with swine, 5 times with bovine) and similar results have been obtained in all cases. In 3 of 14 cases, symptoms reappeared within 48 hours and a second IM administration was carried out. Following this second treatment, symptoms disappeared and no further treatments were needed.

### EXAMPLE 4 - BOVINE TREATMENT

In 2005, a ranching operation calved 264 cows. During that year, 7 calves were lost due to scours, 6 were lost due to pneumonia and 9 died from both diseases. This totalled 22 of 264 calves born with a 8.3% mortality rate.

In 2006, the mineral supplement supplied to the cows was altered such that the mineral was approximately 18-20% of the equilibiotic compound as discussed above. Subsequently, of 245 cows calved, only two died and that was due to physical injury during a storm, not due to a microbial infection.

### EXAMPLE 5- SHEEP TREATMENT

In 2006 a 120 ewes to lamb operation incurred the following problems. The sheep had foot rot, scours, pneumonia, arthritis and general overall bad health with losses of 23 of the 186 lambs born. The most significant concern was that over 25% (ie. 43 ewes) suffered from chronic arthritis in the joints of 2 or more legs. This resulted in a large cost for treatment and handling. The treatment used in 2006 was antibiotics, Ivomec, 6 way vaccine, selenium, and vitamin E. In 2007, 120 ewes lambed in March. There were no scours, no pneumonia in the lambs. In the ewes, there were no arthritis issues, no pregnancy disease, no pneumonia or micoplasma coughing, negligible foot rot and far less cold weather stress. Overall flock health was considered to be excellent and much improved in 2007. The lamb death was reduced from over 10% in 2006 to less that 5% (ie. 5 lambs) in 2007. The only treatment used in 2007 was properly administered equilibiotic compound at 50 lbs per ton in feed ration.

### EXAMPLE 6-SWINE TREATMENT OF PCV 2

PCV 2 is a circa virus-wasting disease in swine. A controlled trial was conducted with a farrow to finish operation of swine. 60 weaners received equilibiotic compound compared to a 120 weaner group which were vaccinated for the virus. After 4 weeks there was zero death loss in the equilibiotic compound group with 2 deaths in the vaccinated group. It is noted that normally, without any treatment over 20% of animals infected with this virus would die. The observations and conclusions drawn by the farmer-producer were in favour of the equilibiotic compound over the vaccination program. Growth rates and overall health were obviously better according to the producer with the administration of the equilibiotic compound.

### EXAMPLE 7- MUMMYFIED NEWBORN PIGLETTS

A 500 sow farrow to finish operation recorded 25 mummyfied baby piglets for every 26 farrowings. After the proper administration of the equilibiotic compound there were only 5 to 6 mummyfied baby piglets for every 26 farrowings. In the producers words, "this is hugely significant". The administration levels were 7.5kg per 1000 kilograms of total feed ration.

### EXAMPLE 8-LUNG AND RESPIRATORY DISORDERS IN HUMANS

A human male at the age of 50 with a compromised immune system due to a teenage spleenectomy suffered from chronic pneumonia. From the age of 39 to the age of 47 years this male individual would end up in the hospital on the average of 2.3 times per year to receive intravenous electrolyte re-hydration and massive amounts of antibiotic treatment. This man started taking three 650 milligram capsules orally of the equilibiotic compound on a daily basis. There has been no re-occurrence.

### EXAMPLE 9- BONE JOINT AND MUSCLE DISORDERS IN HUMANS

An elderly lady at the age of 72 was formerly diagnosed and suffered from osteoarthritis, rheumatoid arthritis and gout and fibromyalgia. She was being treated with Celebrex, a non steroidal anti-inflammatory drug (NSAID).

After 2 years of treatment, her hand and toe joints had nodules and her attaching bones were crooked. She had braces on both wrists and had great trouble walking, continually reporting a squeezing pain in her arms and legs.

Furthermore, she was encountering kidney failure. On her own accord in July 2006 she discontinued the Celebrex treatment. By February 2007, the nodules on both her hands and feet had visible reduced by 40% and she was fully mobile once again. She reported her pain level at a 2 to 4 (with 0 being painless and 10 being unbearable pain). Previously, on Celebrex, she reported her pain level from a 5 to a 6.

Furthermore, her attending physician reported to her that her kidney function had improved from 20% to 75% as determined by lab blood and urine tests in February of 2007. This 72 year old lady weighs approximately 170 lbs and her daily dosage of the equilibiotic compound was three 650 milligram oral capsules daily.

### EXAMPLE 10- DIGESTIVE DISORDERS

A 62 year old lady suffering from a peptic ulcer had undergone 3 surgeries to correct her colony of stomach ulcers. The surgeries were unsuccessful as were a broad spectrum of different antibiotic treatments.

Because of her colony of bleeding peptic ulcers, she required monthly blood transfusions. This lady weighing 195 lbs went on four 650 milligram oral capsules of the equilibiotic compound daily starting September 15, 2006. By January 15, 2007 up to June 4, 2007 she has had complete stoppage of her bleeding peptic ulcer and no need of blood transfusions. During this time period, she took no other treatment for her peptic ulcers. Moreover, she reports no discomfort or pain and an endoscopic examination reports no ulcers on the lining of the stomach or the rest of the gastrointestinal tract.

### EXAMPLE 11- HORMONAL DISORDERS IN HUMANS

A 47 year old male suffering from Diabetes Mellitus type 1 had a 3 month blood glucose average reading of 10.5mg/dL (mmol/L). This individual reported polyuria, polydypsia, excessive hunger, blurred vision, drowsiness, nausea and decreased endurance during exercise. His breathing often became deep and rapid and the smell of ketones on his breath resembled nail polish remover. This individual had watched his father die of the same disease while taking insulin therapy. He concluded, for himself, that he was not going to go on insulin therapy unless he absolutely had to. As of August 1, 2007 he finally agreed with his attending physician that unless something changed with his Type 1 diabetes that he would start insulin therapy November 1, 2007. He started taking three 650mg of the equilibiotic compound oral capsules daily on August 1, 2007. When reporting back with his attending physician in the week of November 1, 2007, he startled his doctor with his latest three month blood glucose average down to 8.2mg/dL (mmol/L). He also reported physical symptom relief of the aformentioned type 1 diabetic maladies. Subsequently, on his next three month average his blood glucose level dropped to 6.0 mg/dL (mmol/L). This is considered to be a non-diabetic state. Furthermore, he personally reported feeling great for the first time in years and had none of the above mentioned type 1 Diabetes symptoms. All vectors remain in a non-diabetic state and the individual reports feeling the best he has felt in over a decade.

A 54 year old man was diagnosed as being type 2 diabetic in the fall of 2006 with blood glucose levels of 8.4 on a thirty day average. After taking 600mg of the said equilibiotic compound on a daily basis for 90 days another sample of blood was taken. The blood glucose level dropped to 5.1. This person also experienced a simultaneously lowering of cholesterol from 3.48 to 3.00.

### EXAMPLE 12-CANCER IN HUMANS

A 71 year old male was diagnosed with gastrointestinal (GI cancer) in the summer of 2004. In the summer of 2004 to the summer of 2006 he was treated unsuccessfully with chemotherapy. His cancer specialist recommended radiation therapy. This individual, on his own accord decided against radiation therapy. He was told by his cancer specialist based on computed aided tomography (CT) and ultrasound that his chances of remission were for all intensive purposes nil. He decided to go on large levels of prescribed pain killers and die at home. Treatment with the equilibiotic compound at three 650mg of oral capsules daily began on July 16, 2006. This individual reported an extreme reduction in abdominal pain immediately within 24 hours.

Furthermore, on the third day he enjoyed a full bowel movement unlike any other experienced in the last two years of his life. Within two weeks he was able to withdraw from pain killers and once again became active with his family. In March of 2007, he underwent a CT and an ultrasound. His attending physician, a cancer specialist, was astounded and reported a significant clearance of GI cancer. As of this date, June 4, 2007, this 71 year old male reports a happy and healthy lifestyle with his family.

### EXAMPLE 13-AUTOIMMUNE DISORDER IN HUMANS

A 55 year old man who had suffered Lupus for several years orally ingested the said equilibiotic compound at a rate of 650mg per day commencing in September 2006. After 45 days, blood tests were done and it was determined that a problem associated with Lupus urea in the urine of the patient had been corrected. The only change in medication and diet for the patient was the addition of the said equilibiotic compound in the diet. Since taking the said equilibiotic compound on a daily basis, the patient to this date has enjoyed unusually good health considering his illness.

### EXAMPLE 14- VIRAL INFECTIONS IN HUMANS

A male 67 years of age had suffered from Herpes simplex virus-2 (HSV- 2). HSV-2 cause genital herpes. For over 30 years this male individual suffered from HSV-2 infections producing an eruption of tiny blisters on his skin and mucus membranes. For over 30 years these eruptions of blisters lasted from 10-15 days. These genital HSV-2 infections were severe and prolonged with multiple painful blisters in the genital area. This virus caused fever, burning during urination, and great groin discomfort and itching. It was concluded after 30 years of antiviral treatments they were unsuccessful in eradicating the HSV-2 infection. However, treatment at three 650mg oral capsule daily with the said equilibiotic compound gave discomfort relief and shortened the duration of the outbreak from 10 to 15 days to 1 to 2 days with continuous therapy. Moreover, the outbreaks were only mere redness of the skin in the genital area with no open sores.

### EXAMPE 15-SKIN DISEASES IN HUMANS

A 60 year old man suffering from psoriasis on 2/3 of his body had been on a disability pension for a number of years. After taking the said equilibiotic compound at the rate of approximately 1 gm per day for several weeks, he began to experience some symptomatic relief from his condition.

After continuing to the said equilibiotic compound at the same rate for a year, almost all of the psoriasis infected areas have cleared up with only a small amount remaining on his arms. It is anticipated that he well recover totally from this debilitating disorder.

### EXAMPLE 16-SKIN DISEASES IN POULTRY

A poultry producer fed the said equilibiotic compound at the rate of 1% of ration to 7000 broilers over a period of 42 days. The purpose of the trial was to see if the incidence of cellulites could be reduced in the flock when compared to other flocks being fed and marketed simultaneously from the same farm. After slaughter, it was found that the cellulites was reduced by 67% compared to the control flocks.

### EXAMPLE 17-SEXUAL AND REPRODUCTIVE DYSORDERS IN HUMANS

A woman reached early menopause at the age of 27 years. She suffered from hypothyroidism (Hashimoto's). Hypothyroidism is an under-activity of the thyroid gland. She was treated with Eltroxin, a form of thyroid hormone replacement therapy.

Menopause is usually a permanent and of cyclic functioning of the ovaries and thus of menstrual periods. This particular woman began to cycle normally after 16 years of menopause with the proper administration of the said equilibiotic compound. At the age of 43, in the month of November of 2006 she began orally taking four 650mg capsules of the said equilibiotic compound. Normal cyclic functioning of the ovaries and thus of the menstrual periods began immediately in the subsequent month of November 2006. As of June 2007, normal sexual cycling and functioning continues. This woman no longer suffers from the menopausal symptoms of mood changes, depression, irritability, head ache, insomnia and fatigue.

## Claims

1. A pharmaceutical composition comprising as an active ingredient sulphur particles less than 300 microns and sodium lignin sulfonate, for use in the treatment of lung and respiratory disorders, digestive disorders, diabetes, cancer and/or viral infections, wherein the composition is to be administered orally or by injection.

2. A pharmaceutical composition consisting of as an active ingredient sulphur particles less than 300 microns and sodium lignin sulfonate, for use in the treatment of lung and respiratory disorders, digestive disorders, diabetes, cancer and/or viral infections, wherein the composition is to be administered orally or by injection.

3. The pharmaceutical composition according to claim 1 or 2 wherein the sulphur particles are less than 75 microns.

4. The pharmaceutical composition of any one of claims 1 to 3 wherein 95-98% of the sulphur particles are less than 45 microns.

5. A method of preparing the pharmaceutical composition of any one of claims 2 to 4 consisting of admixing an effective amount of sulphur particles less than 300 microns with sodium lignin sulfonate.

6. The method according to claim 5 wherein the sulphur particles are less than 75 microns.

7. The method according to claim 5 wherein 95-98% of the sulphur particles are less than 45 microns.

## Patentansprüche

1. Pharmazeutische Zusammensetzung, die als einen aktiven Inhaltsstoff Schwefelpartikel mit weniger als 300 Mikrometern und Natrium-LigninSulfonat umfasst, zur Verwendung in der Behandlung von Lungen- und Atemwegserkrankungen, Verdauungsstörungen, Diabetes, Krebs und/oder viralen Infektionen, wobei die Zusammensetzung oral oder per Injektion zu verabreichen ist.

2. Pharmazeutische Zusammensetzung, die aus einem aktiven Inhaltsstoff Schwefelpartikel mit weniger als 300 Mikrometern und Natrium-LigninSulfonat besteht, zur Verwendung in der Behandlung von Lungen- und Atemwegserkrankungen, Verdauungsstörungen, Diabetes, Krebs und/oder viralen Infektionen, wobei die Zusammensetzung oral oder per Injektion zu verabreichen ist.

3. Pharmazeutische Zusammensetzung nach Anspruch 1 oder 2, wobei die Schwefelpartikel weniger als 75 Mikrometer groß sind.

4. Pharmazeutische Zusammensetzung nach einem der Ansprüche 1 bis 3, wobei 95-98% der Schwefelpartikel weniger als 45 Mikrometer groß sind.

5. Verfahren zur Herstellung der pharmazeutischen Zusammensetzung nach einem der Ansprüche 2 bis 4, bestehend aus dem Mischen einer wirksamen Menge von Schwefelpartikeln mit weniger als 300 Mikrometern mit Natrium-Lignin-Sulfonat.

6. Verfahren nach Anspruch 5, wobei die Schwefelpartikel weniger als 75 Mikrometer groß sind.

7. Verfahren nach Anspruch 5, wobei 95-98% der Schwefelpartikel weniger als 45 Mikrometer groß sind.

## Revendications

1. Composition pharmaceutique comprenant en tant que principe actif des particules de soufre de moins de 300 microns et du lignosulfonate de sodium, pour une utilisation dans le traitement de troubles respiratoires et des poumons, de troubles digestifs, du diabètes, d'un cancer et/ou d'infections virales, dans laquelle la composition est destinée à être administrée oralement ou par injection.

2. Composition pharmaceutique consistant en, en tant que principe actif, des particules de soufre de moins de 300 microns et du lignosulfonate de sodium, pour une utilisation dans le traitement de troubles respiratoires et des poumons, de troubles digestifs, du diabètes, d'un cancer et/ou d'infections virales, dans laquelle la composition est destinée à être administrée oralement ou par injection.

3. Composition pharmaceutique selon la revendication 1 ou 2 dans laquelle les particules de soufre sont de moins de 75 microns.

4. Composition pharmaceutique selon l'une quelconque des revendications 1 à 3 dans laquelle 95-98% des particules de soufre sont de moins de 45 microns.

5. Méthode de préparation de la composition pharmaceutique selon l'une quelconque des revendications 2 à 4 consistant en le mélange d'une quantité efficace de particules de soufre de moins de 300 microns avec du lignosulfonate de sodium.

6. Méthode selon la revendication 5 dans laquelle les particules de soufre sont de moins de 75 microns.

7. Méthode selon la revendication 5 dans laquelle 95-98% des particules de soufre sont de moins de 45 microns.
